# EUROPEAN PATENT APPLICATION

(11) **EP 2 132 978 A1**
(43) Date of publication of application: **16.12.2009**
(21) Application number: 08010799.8
(22) Date of filing: 13.06.2008
(51) Int. Cl.: A01H 4/00, A01H 5/02, C12N 5/14

(54) **Method for the production of somatic embryos from Euphorbia pulcherrima plant tissue**

(71) Applicant: Syngenta Participations AG, 4058 Basel (CH)
(72) Inventor: Hunold, Reiner, 56237 Deesen (DE); Zerr, Katharina, 56203 Höhr-Grenzhausen (DE)
(74) Representative: Kock, Michael Andreas

(57) **Abstract**

The present invention relates to a method for the production of somatic embryos from poinsettia (*Euphorbia pulcherrima*) plant tissue. Furthermore, the present invention discloses subject matter that relates to poinsettia plants, particularly to non-chimeric poinsettia plants, and to a method of producing the same.

## Description

### Technical Field

The presently disclosed subject matter of this invention relates to a method for the production of somatic embryos from poinsettia (*Euphorbia pulcherrima*) plant tissue. Furthermore, the present invention discloses subject matter that relates to poinsettia plants, particularly to non-chimeric poinsettia plants, and to a method of producing the same.

### Background

The poinsettia, *Euphorbia pulcherrima*, is a member of the family *Euphorbiaceae* and has become one of the most important ornamentals worldwide and is the primary potted flowering plant produced and sold in North America. The combined market volume of poinsettia in the US and Europe is around 500 Million Euro each year, which makes the production of poinsettia potted plants to an important component of the floral industry. The genus *Euphorbia* is characterized by a single female flower that typically lacks petals and sepals. The most prominent feature of the poinsettia plants is the bright color of the bract, modified leaves popularly referred to as "flowers". The commercial importance of a poinsettia plant is mainly determined by its bract color, and, therefore the production of plants with new and consumer attractive bract colors is one of the most important breeding goals for the poinsettia breeder. Although the genus *Euphorbia* contains more than 700 species, the color range of the bract is mainly limited to red, white, yellow, pink or mixtures thereof. The internet page of the National poinsettia Cultivar Trials (http://flowers.hort.purdue.edu/poinsettiaSite/default.html) provides an comprehensive overview of the commercially available poinsettia varieties and their characteristics. Said internet page contains a freely accessible database covering information related to poinsettia cultivar trials from 1998 to 2005, provided from universities and commercial breeders. The bract colors of the different poinsettia varieties listed in the mentioned database is rather limited ranging from white, red, pink, burgundy, coral, cranberry, marble, mauve, rose and salmon, whereby 70-75% of said varieties possess bracts with red color. Since the commercial success of poinsettia varieties depends mostly on an attractive bract color, it is desirable to produce poinsettia plants with an unique, preferably orange-like bract color not found in the prior art so far.

In the literature it is described that the bract color of the poinsettia plants is determined by a single dominant gene. Plants homozygous for the recessive allele develop anthocyanin free (white) bracts. The origin of bract color variations are determined by the appearance of periclinal chimeras lacking anthocyanins in the L1 histogenic layer (Preil, W. 1986. In vitro propagation and breeding of ornamental plants: advantages and disadvantages of variability. In W. Horn, C.J. Jensen, W. Odenback, and O. Schieder (eds), Genetic Manipulation in Plant Breeding, proceedings of an international symposium organized by EUCARPIA, Sept. 8-13, 1985, Berlin (West), Germany, pp. 377-403. Walter de Gruyter & Co., N.Y.). Most poinsettia cultivars available on the market are periclinal chimeras produced via mutation breeding approaches. Said varieties are relatively stable and can be propagated vegetatively. A mutation produces a periclinal chimera if the affected meristematic cell is positioned near the apical dome so that the cells produced by subsequent divisions form an entire layer of the mutated type. The resulting meristem contains one layer which is genetically different from the remainder of the meristem. In poinsettia 3 histogenic layers are recognized L1-epidermis, L2-palisade and spongy parenchyma and L3-inner spongy parenchyma. Chimeric plants with mutations in the L1 layer have the disadvantage that their specific phenotype will not be transferred sexually into the next generation. Hence, said plants can not be used for breeding purposes and have to be propagated vegetatively, which in turn can lead again to the induction of additional mutations. Many of these mutations remain undetected and, therefore, are so called "crypto-chimeras". An additional problem of the chimeric plants is the fact that the specific color is the result of the combination and interaction of genetically different histongenic cell layers. Hence, care has to be taken to prevent cell layer rearrangements during propagation, which might lead to the reversion (back-mutation) of the phenotype. The frequency of back mutations, resulting in plants that can not be sold, is between 2 and 20% depending on the genetic background and the culture conditions. In order to avoid back mutations and the propagation of back mutated plants, the breeder has to select the elite plants used for the mass propagation permanently. This generates additional cost and effort for the breeder. Hence, the preparation of non-chimeric plants, particularly of plants with new colors not yet available on the market, is highly desirable.

In vitro culture is one of the key tools of plant biotechnology that exploits the totipotency nature of plant cells, a concept proposed by Haberlandt (Haberlandt G. Kulturversuche mit isollierten pflanzenzellen. S.B. Weisen Wien Naturwissenschaften, vol. 111. 1902. p. 69-92) and unequivocally demonstrated, for the first time, by Steward et al. (Steward FC, Mapes MO, Mears K. Growth and organised development of cultured cells: II. Organisation in cultured grown from freely suspended cells. Am J Bot 1958;45:705-7.) Ornamental industry has applied immensely *in vitro* propagation approaches for largescale plant multiplication of elite superior varieties. About 156 ornamental genera are propagated through tissue culture in different commercial laboratories worldwide. The identification of chimeric or crypto-chimeric poinsettia plants by using *in vitro* culture techniques has been described too (W.Preil und Margarete Engelhardt, 1982, In vitro-Entmischung von Chimärenstrukturen durch Suspensionskulturen bei Euphorbia pu/cherrima, Willd., Gartenbauwissenschaft, 47 (6), S. 241-244). One of the advantages of somatic embryogenesis compared to suspension cultures, axillary or adventitious bud propagation techniques, is that embryos are bipolar structures with functional shoot and root meristem. There is, thus, no need to cut and root the individual organ. Furthermore, by using somatic embryogenesis, virus infection or other pathogens present in the plant or in plant tissues/organs can be eliminated completely or substantially reduced. However, the above mentioned poinsettia cell culture technique for the regeneration of whole plants out of single cells is only applicable for a limited number of poinsettia varieties. Hence, the existing methods have only been used to provide a qualitative proof that some varieties are of chimeric nature (W.Preil und Margarete Engelhardt, 1982, In vitro-Entmischung von Chimärenstrukturen durch Suspensionskulturen bei Euphorbia pulcherrima, Willd., Gartenbauwissenschaft, 47 (6), S. 241-244) or to regenerate cells out of a cells suspension culture treated with mutagenic agents (F. Walther and W. Preil, Mutants tolerant to low temperature conditions induced in suspension culture as a source for improvement of Euphorbia pulcherrima Willd. Ex. Klotzsch. IAEA, Vienna, 1981, 399-405). Although in 1999 Osternack et al. (Osternack N, Saare-Surminski K, Preil W, Lieberei R. Induction of somatic embryos, adventitious shoots and roots in hypocotyls tissue of Euphorbia pulcherrima Willd. Ex Klotzsch: comparative studies on embryogenic and organogenic competence. J Appl Bot 1999;73:197-201) succeeded in inducing somatic embryogenesis from hypocotyl tissues of *Euphorbia pulcherrima* on MS medium supplemented with 2.0 mg/l IAA, only 8% of the embryos produced by said method developed normal plantlets. Furthermore, said method does not work for all *Euphorbia pulcherrima* varieties. An improved method for the production of somatic embryos by using non-Daucus non-callus explant material in combination with liquid culture medium has been disclosed in the patent EP 0 608 716 B1. However, said patent does not disclose a method for the production of somatic embryos using poinsettia plant tissue.

It was therefore an objective of the present invention to provide a new method for the production of somatic embryos from poinsettia plant tissue, which does not have the above mentioned drawbacks of the prior art methods. Particularly, it was an objective of the invention to provide a method for the production of somatic embryos that can be applied to a wider range of *Euphorbia pulcherrima* varieties and that has a greater efficiency compared to the prior art methods. Additionally, it was an objective of the present invention to provide a method for the production of non-chimeric *Euphorbia pulcherrima* plants, particularly of *Euphorbia pulcherrima* plants having a new and distinct bract color.

### SUMMARY OF THE INVENTION

The present disclosure features a method for the production of somatic embryos from poinsettia plant tissue, **characterized in that** an embryo induction medium is used comprising between at least 0.1 and 1 mg/l a-naphthalene acetic acid (NAA) and between at least 0.2 and 1 mg/l Thidiazuron, with the proviso that if the α-naphthalene acetic acid concentration is below 0.2 mg/l the Thidiazuron concentration is at least 0.4 mg/l. The method can include the use of young shoot tissue. In one aspect of the invention, the plant tissue used for the production of the somatic embryos is derived from a poinsettia plant selected from the group consisting of the varieties Cortez Eletric Fire (US PP 17088), "Fissilver" (US Plant Patent PP9989), "Nobelstar" (US Plant Patent PP9474), "Fisflirt" (US Plant Patent PP9385), "Fispic" (US Plant Patent PP9371), "Fisbon" (US Plant Patent PP9347), "Fisbla" (US Plant Patent PP9316), "Fispue" (US Plant Patent PP9315), "Fismars Pink" (US Plant Patent PP18866), "Fisdra" (US Plant Patent PP17739), "Fismars Creme" (US Plant Patent PP17658), "Fiscinne" (US Plant Patent PP16964), "Fisholly" (US Plant Patent PP16945), "Fisnovired" (US Plant Patent PP16869), "Fisvinci" (US Plant Patent PP14107), "Kamp Burgundy" (US Plant Patent PP13962), "Fislemon" (US Plant Patent PP13747), "Fiselfi" (US Plant Patent PP13736), "Fisson Jinglit" (US Plant Patent PP13721), "Fisson Beckpink" (US Plant Patent PP13717), "Fismille" (US Plant Patent PP13660), "Fispue White" (US Plant Patent PP13659), "Fismarble Silver" (US Plant Patent PP13174), "Fisson Jingle" (US Plant Patent PP12540), "Fisson Piz" (US Plant Patent PP12539), "Fiscor Hot Pink" (US Plant Patent PP12501), "Fisson Orange" (US Plant Patent PP12500), "Fisnova" (US Plant Patent PP12387), "Fisson Gold" (US Plant Patent PP12298), "Fisgala" (US Plant Patent PP12178), "Fiscor Candy" (US Plant Patent PP11593), "Fisflirt Silver" (US Plant Patent PP11585), "Fisson Marble" (US Plant Patent PP10835), "Fisson White" (US Plant Patent PP10825), "Fiscor Creme" (US Plant Patent PP10824), "Fiswhite silver" (US Plant Patent PP14626), "Fiscor Fire" (US Plant Patent PP12992), "Fiscor dark red" (US Plant Patent PP12723), "Fismond" (US Plant Patent PP14110), "Fismars" (US Plant Patent PP14997), "Fisolymp" (US Plant Patent PP14662), "Fisson dark red" (US Plant Patent PP12722), "Fiselfy pink" (US Plant Patent PP14899), "Fisson" (US Plant Patent PP9365), "Fiscor" (US Plant Patent PP9364), "Fissonosa" (US Plant Patent PP10182) and "Fiscorosa" (US Plant Patent PP10077).

In a further preferred embodiment, the plant tissue used for the production of the somatic embryos can also be derived from any other commercially available poinsettia plant.

The invention furthermore encompasses growing mature plants from the somatic embryos produced according to the above described method.

In one aspect, the present invention provides methods for producing a non-chimeric poinsettia plant comprising the steps of (a) producing somatic embryos as described above and (b) growing a mature plant from a somatic embryo produced in step a), **characterized in that** the poinsettia plant used in step a) is a chimeric plant. In an additional aspect of the invention, the chimeric poinsettia plant used in step (a) described above has been produced by mutational breeding. In another aspect of the invention, the method for the production of non-chimeric poinsettia plants comprises the additional step of determining the non-chimeric status of the mature plants produced according to said method.

The present invention encompasses further a method for the production of chimeric or non-chimeric poinsettia plants out of somatic embryos produced as described above, additionally comprising the step of infecting the produced plants with a phytoplasma in order to induce free branching.

In another aspect, the phytoplasma infection is achieved by grafting shoots of the plants produced as described above on an infected rootstock or by using approach grafting.

The present invention encompasses non-chimeric Pionsettia plants, **characterized in that** the intercostals areas of the bracts have a colour ranging from 30A to 40A measured with reference to the colour patch of The Royal Horticulture Society Colour Chart and the progeny thereof.

In another aspect, the invention relates to an embryo induction medium comprising between at least 0.1 and 1 mg/l a-naphthalene acetic acid (NAA) and between at least 0.2 and 1 mg/l Thidiazuron, with the provisio, that, if the a-naphthalene acetic acid concentration is below 0.2 mg/l, the Thidiazuron concentration is at least 0.4 mg/l.

Further aspects and features of the invention will be apparent upon inspection of the following detailed description thereof.

### GENERAL DEFINITIONS

It must be noted that as used herein and in the appended claims, the singular forms "a" and "the" include plural reference unless the context clearly indicates otherwise. Thus, for example, reference to "a plant" is a reference to one or more plants and includes equivalents thereof known to those skilled in the art.

"Chimeric" in the context of "a chimeric plant" refers to a poinsettia plant or a part thereof made up of two or more genetically distinct cells of the same kind (chimerism). "Genetically distinct" in respect to cells, refers to cells that, although belonging to the same cell type, or being located in the same tissue or organ, differ in the nucleic acid sequence of at least one gene, or an nucleic acid element controlling the expression of said gene, whereby said difference leads to a visible phenotypic difference between said cells or plant parts, tissues or organs made up of said cells. Preferably, the difference in the nucleic acid sequence leads to a visible difference in the bract color of the poinsettia plants. The difference in the nucleic acid sequence can be the result of an induced or spontaneous mutation. A chimeric plant can arise when a cell of said plant undergoes mutation. Said chimeric plants may originate by spontaneous-mutation or it may be induced by irradiation or treatment with chemical mutagens. If the cell which mutates is located near the apical dome, then all other cells which are produced there from will be of the mutated type. The result will be a tissue comprising cells of different genotypes. Chimeras can be classified as periclinal, mericlinal or sectorial chimeras. A mutation produces a periclinal chimera if the affected cell is positioned near the apical dome so that the cells produced by subsequent divisions form an entire layer of the mutated type. The resulting meristem contains one layer which is genetically different from the remainder of the meristem. Mericlinal chimeras are produced when the derivatives of the mutated cell do not entirely cover the apical dome. A mutated cell layer may be maintained on only one portion of the meristem giving rise to chimeral shoots or leaves which develop in that portion while those that differentiate on all other portions of the meristem are normal, non-chimeral shoots. Many mericlinal chimeras involve such a limited number of cells that only a small portion of one leaf may be affected. As was the case with periclinal chimeras, mericlinal chimeras are generally restricted to one cell layer. Sectorial chimeras result from mutations which affect sections of the apical meristem, the altered genotype extending through all the cell layers. This chimeral type is unstable and can give rise to shoots and leaves which are not chimeras.

"Non-chimeric" in respect to plants, refer to plants that are made up of cells that are genetically identical in the sense that their genetic information does not give rise to cells or plant parts that are phenotypically distinct from other cells or plant parts of the same tissues or organ. Preferably, the non-chimeric plants expressing a uniform bract color.

As used herein, the term "color" refers to the normal, everyday meaning of the word. As regards the color in reference to a plant the term includes the overall color(s) of a plant or plant part as well as the gradations in color(s) of a plant or plant part, including color patterning. Thus, in the context of this invention, the term "color" when referring to a poinsettia plant includes the colors associated with leafs, particularly with the bracts. The color of any particular plant or plant part, or variations in the color thereof, may be referenced to color designations set forth in a recognized color dictionary. Examples of such dictionaries include but are not limited to the Munsell Book of Color and the Royal Horticulture Society Colour Chart (RHSCC). The RHSCC is the standard reference for plant colour identification and can be purchased from The Royal Horticulture Society (http://www.rhs. org.uk/ publications/pubs_library_colourchart.asp)

"Embryo induction medium" refers to a cultivation medium used in the somatic embryogenesis process, which allows for the culturing and production of mature somatic embryos.

Phytoplasma refers to very small prokaryotes that do not have a cell wall and that are found in the phloem cells of host plants, and are normally considered as plant pathogens. The infection with Phytoplasma is desirable in poinsettia as it causes poinsettia to produce more branches (Ing-Ming Lee et al., Phytoplasma induced free-branching in commercial poinsettia cultivars, Nature Biotechnology 15, 178 - 182 (1997))

The phrase "grafting on an infected rootstock" or the term "approach grafting" as used herein, refers to a piece of tissue from a plant (e.g. shoot), implanted into/onto another plant-tissue or -organ (e.g. rootstock). Grafting methods are well known in the art.

As used herein, the term "progeny" refers to asexually propagated cuttings as well as to sexually propagated filial generation of the particular parental generation.

As used herein, the terms "mutant" or "mutation" refer to a gene, cell, or organism with an abnormal genetic constitution that may result in a variant phenotype.

As used herein, the terms "nucleic acid" or "polynucleotide" refer to deoxyribonucleotides or ribonucleotides and polymers thereof in either single- or double-stranded form.

As used herein, the terms "phenotype" or "phenotypically refer to the observable characters of an individual cell, cell culture, organism (e.g., a plant), or group of organisms which results from the interaction between that individual's genetic makeup (i.e., genotype) and the environment.

As used herein, the term "plant tissue" refers to any part of a plant. Examples of plant organs include, but are not limited to the leaf, stem, root, seed, branch, nodule, leaf axil, flower, pollen, stamen, pistil, petal, stalk, stigma, bract, carpel, sepal, anther, and ovule.

As used herein, the term "tissue culture" refers to a technique, process or method of keeping tissue alive and growing in a culture medium. The term may also refer to the actual culture of tissue grown using these techniques, processes or methods

By "somatic embryogenesis" is meant a process of embryo initiation and development from vegetative or non-gametic cells, that are not normally involved in the development of embryos.

### DETAILED DESCRIPTION OF THE INVENTION

The teaching of the present invention enables the production of non-chimeric *Euphorbia pulcherrima* plants by overcoming drawbacks of the current prior art methods for the production of somatic embryos from *Euphorbia pulcherrima* plant tissue.

A first embodiment of the invention relates to a method for the production of somatic embryos from poinsettia plant tissue, **characterized in that** an embryo induction medium is used comprising between at least 0.1 and 1 mg/l a-naphthalene acetic acid (NAA) and between at least 0.2 and 1 mg/l Thidiazuron, with the proviso that if the α-naphthalene acetic acid concentration is below 0.2 mg/l the Thidiazuron concentration is at least 0.4 mg/l.

In a preferred embodiment, the embryo induction medium used in the inventive method comprises between at least 0.1 and 1 mg/l α-naphthalene acetic acid (NAA) and between at least 0.4 and 1 mg/l Thidiazuron, particularly preferred is an embryo induction medium comprising between 0.1 and 0.4 mg/l a-naphthalene acetic acid (NAA) and 0.4 mg/l Thidiazuron. In a most preferred embodiment, the embryo induction medium comprises 0.1 mg/l α-naphthalene acetic acid (NAA) and 0.4 mg/l Thidiazuron.

In a furthermore preferred embodiment, young shoot tissue is employed for the production of somatic embryos. Preferably, shoot tips of at least 1,5 cm length were excised from plants and sterilized. In a particularly preferred embodiment, subapical stem segments having a thickness between at least 1 and 5 millimetre, preferably between 1 and 3 mm are taken from said sterilized axillary shoots and used for the induction of somatic embryogenesis and for further development of embryogenic callus.

In a furthermore preferred embodiment of the invention, the tissue that is used for the production of the somatic embryos is taken from a poinsettia plant that is selected from the group consisting of the varieties Cortez Eletric Fire (US PP 17088), "Fissilver" (US Plant Patent PP9989), "Nobelstar" (US Plant Patent PP9474), "Fisflirt" (US Plant Patent PP9385), "Fispic" (US Plant Patent PP9371), "Fisbon" (US Plant Patent PP9347), "Fisbla" (US Plant Patent PP9316), "Fispue" (US Plant Patent PP9315), "Fismars Pink" (US Plant Patent PP18866), "Fisdra" (US Plant Patent PP17739), "Fismars Creme" (US Plant Patent PP17658), "Fiscinne" (US Plant Patent PP16964), "Fisholly" (US Plant Patent PP16945), "Fisnovired" (US Plant Patent PP16869), "Fisvinci" (US Plant Patent PP14107), "Kamp Burgundy" (US Plant Patent PP13962), "Fislemon" (US Plant Patent PP13747), "Fiselfi" (US Plant Patent PP13736), "Fisson Jinglit" (US Plant Patent PP13721), "Fisson Beckpink" (US Plant Patent PP13717), "Fismille" (US Plant Patent PP13660), "Fispue White" (US Plant Patent PP13659), "Fismarble Silver" (US Plant Patent PP13174), "Fisson Jingle" (US Plant Patent PP12540), "Fisson Piz" (US Plant Patent PP12539), "Fiscor Hot Pink" (US Plant Patent PP12501), "Fisson Orange" (US Plant Patent PP12500), "Fisnova" (US Plant Patent PP12387), "Fisson Gold" (US Plant Patent PP12298), "Fisgala" (US Plant Patent PP12178), "Fiscor Candy" (US Plant Patent PP11593), "Fisflirt Silver" (US Plant Patent PP11585), "Fisson Marble" (US Plant Patent PP10835), "Fisson White" (US Plant Patent PP10825), "Fiscor Creme" (US Plant Patent PP10824), "Fiswhite silver" (US Plant Patent PP14626), "Fiscor Fire" (US Plant Patent PP12992), "Fiscor dark red" (US Plant Patent PP12723), "Fismond" (US Plant Patent PP14110), "Fismars" (US Plant Patent PP14997), "Fisolymp" (US Plant Patent PP14662), "Fisson dark red" (US Plant Patent PP12722), "Fiselfy pink" (US Plant Patent PP14899), "Fisson" (US Plant Patent PP9365), "Fiscor" (US Plant Patent PP9364), "Fissonosa" (US Plant Patent PP10182) and "Fiscorosa" (US Plant Patent PP10077). In a preferred embodiment, the plant tissue used for the production of the somatic embryos is taken from a poinsettia plant selected from the group consisting of the varieties Fiscor Electric (US PP 17088), "Fiselfi" (US Plant Patent PP13736), "Fisson" (US Plant Patent PP9365), "Fiscor" (US Plant Patent PP9364), "Fissonosa" (US Plant Patent PP10182), "Fismars" (US Plant Patent PP14997), "Fiscorosa" (US Plant Patent PP10077) and "Fisdra" (US Plant Patent PP17739).

In yet another alternative embodiment of the invention, the somatic embryos produced according to the inventive method are further cultivated in order to produce mature poinsettia plants.

The invention relates furthermore to an embryo induction medium comprising between at least 0.1 and 1 mg/l α-naphthalene acetic acid (NAA) and between at least 0.2 and 1 mg/l Thidiazuron, with the provisio, that, if the a-naphthatene acetic acid concentration is below 0.2 mg/l, the Thidiazuron concentration is at least 0.4 mg/l. In a preferred embodiment, the embryo induction medium comprises between at least 0.1 and 1 mg/l a-naphthalene acetic acid (NAA) and between at least 0.4 and 1 mg/l Thidiazuron, particularly preferred is an embryo induction medium comprising between 0.1 and 0.4 mg/l a-naphthalene acetic acid (NAA) and 0.4 mg/l Thidiazuron. In a most preferred embodiment, the embryo induction medium comprises 0.1 mg/l a-naphthalene acetic acid (NAA) and 0.4 mg/l Thidiazuron.

An additional embodiment of the invention relates to a method for producing a non-chimeric poinsettia plant comprising the steps of:
a) producing somatic embryos according to the above described method, and
b) growing a mature plant from a somatic embryo produced in step a), **characterized in that** the poinsettia plant used in step a) is a chimeric plant.

In a furthermore preferred embodiment of the invention, the tissue that is used for the production of the somatic embryos or the production of non-chimeric poinsettia plants is taken from a poinsettia plant selected from the group consisting of the varieties Cortez Eletric Fire (US PP 17088), "Fissilver" (US Plant Patent PP9989), "Nobelstar" (US Plant Patent PP9474), "Fisflirt" (US Plant Patent PP9385), "Fispic" (US Plant Patent PP9371), "Fisbon" (US Plant Patent PP9347), "Fisbla" (US Plant Patent PP9316), "Fispue" (US Plant Patent PP9315), "Fismars Pink" (US Plant Patent PP18866), "Fiscinne" (US Plant Patent PP16964), "Fisholly" (US Plant Patent PP16945), "Kamp Burgundy" (US Plant Patent PP13962), "Fisson Jinglit" (US Plant Patent PP13721), "Fisson Beckpink" (US Plant Patent PP13717), "Fispue White" (US Plant Patent PP13659), "Fismarble Silver" (US Plant Patent PP13174), "Fisson Jingle" (US Plant Patent PP12540), "Fisson Piz" (US Plant Patent PP12539), "Fiscor Hot Pink" (US Plant Patent PP12501), "Fisson Orange" (US Plant Patent PP12500), "Fisson Gold" (US Plant Patent PP12298), "Fisflirt Silver" (US Plant Patent PP11585), "Fisson Marble" (US Plant Patent PP10835), "Fiswhite silver" (US Plant Patent PP14626), "Fiscor Fire" (US Plant Patent PP12992), "Fiscor dark red" (US Plant Patent PP12723), "Fisolymp" (US Plant Patent PP14662), "Fisson dark red" (US Plant Patent PP12722), "Fiselfy pink" (US Plant Patent PP14899), "Fissonosa" (US Plant Patent PP10182) and "Fiscorosa" (US Plant Patent PP10077). In a preferred embodiment, the plant tissue used for the production of the somatic embryos is taken from a poinsettia plant selected from the group consisting of the varieties "Fissonosa" (US Plant Patent PP10182) and "Fiscorosa" (US Plant Patent PP10077)

In a particularly preferred embodiment of the invention, the chimeric poinsettia plant used for the production of a non-chimeric poinsettia plant -as decribed above- has been produced by mutational breeding. Mutational breeding of poinsettia plants is a routine method and the skilled person is well aware of the different mutagens and conditions that are applicable in the mutational breeding process (e.g. Broertjes et al., Application of Mutation Breeding Methods in the improvement of Vegetatively Propagated Crops, Elsevier Scientific Publishing Company, 1978, pp. 118-119).

Mutations of plants can be induced by standard methods (see, e.g., U.S. Pat. No. 6,484,105; A. M. van Harten, Mutation Breeding: Theory and Practical Applications, 378 pages, Cambridge University Press, 2007). For instance, seeds or other plant material can be treated with a mutagenic chemical substance, e.g., diepoxybutane, diethyl sulfate, ethylene imine, ethyl methanesulfonate and N-nitroso-N-ethylurea. Alternatively, ionizing radiation from sources such as, for example, X-rays, gamma rays or fast neutron bombardment can be used. A mutagenized population is typically developed by generating a large number of plants and exposing said plants to a mutagen. A skilled practitioner will recognize that a series of concentrations and a time of exposures are used to determine the proper level of mutagenesis. Typically, such test populations are planted in soil or plated onto solid growth media. Mutagens can produce, inter alia, point mutations, deletions, inversions, insertions, duplications and rearrangements.

In an additional embodiment of the invention, the above describe method for the production of non-chimeric poinsettia plants comprises an additional step c) in which the non-chimeric status of the mature plants grown and produced in step b) is determined.

Methods for the identification of chimeric and non-chimeric plants are well known in the art. Three general characteristics denote chimeric plants:
1. Somatic segregation: spontaneously as a sport or induced through in vitro callus culture, adventitious shoot formation or somatic embryogenesis, the plants segregate into their unique genetic components (W.Preil und Margarete Engelhardt, 1982, In vitro-Entmischung von Chimärenstrukturen durch Suspensionskulturen bei Euphorbia pulcherrima, Willd., Gartenbauwissenschaft, 47 (6), S. 241-244. and Preil, W., 1994: In vitro culture of poinsettia. In: Stromme, E (ed), The Scientific Basis of poinsettia Production, 49-55. Agric. Univ. of Norway, As),
2. Chimeric patterns are not to remain by sexual reproduction (self-pollination or crossing), and
3. Single layers can be double-marked, for example by differences in anthocyanin synthesis in connection with different ploidy levels, which make it possible to categorize tissues over the whole ontogenesis of the plant to the appropriate apex layers (Pohlheim, F. and Plaschil, S. (2001) Doppelmarkierung von Periklinalchimären-eine Analysenmethode. BDGL-Schriftenreihe 19: 113. and Pohlheim, F. and Rössel, K. 1989. Partnerinduktion bei chimärischen Blatt- und Blütenfarbmustern von Pelargonium. Tag. Ber. Akad. Landwirtsch. DDR 218: 107-115, Berlin.)

Additionaly, a vey simple method for the identification of chimeric plants has been developed already 1916 by W. Bateson (Bateson, W. 1916. Root Cuttings, Chimeras and Sports. Journ. Genet. 6: 75-80.) Said method is based on the induced formation of adventitious shoots from roots and lead to the segregation of the inner component (L3). All shoots were removed near the root collar and some vigorous roots were cultivated under fog conditions to induce adventitious shoots, which, in case of a chimeric plant, give rise to shoots that are phenotypically distinct. Furthermore, the inventive method for the production of somatic embryos disclosed in this patent application can be used to analyse the chimeric or non-chimeric status of a poinsettia plant.

In another preferred embodiment of the present invention, the plants and/or non-chimeric plants that have been cultivated or grown out of somatic embryos produced according to the inventive method are subsequently infected with a phytoplasma in order to induce the free branching of said plants. Preferably, the phytoplasma infection is achieved by grafting shoots taken from plants, or non-chimeric plants, derived from somatic embryos produced according to the inventive method on an infected rootstock or by approach grafting. The distinguishing feature of approach grafting is that two independently growing, self-sustaining plants are grafted together. This self-sustaining characteristic of both plants which are to be grafted insures survival of both plants. Grafting methods and methods for the infection of poinsettia plants with phytoplasma are well known in the art (e.g. US 4,724,276, Process for altering poinsettia growth characteristics)

Additionally, the invention relates to a non-chimeric Pionsettia plant and the progeny thereof, **characterized in that** the intercostals areas of the bracts of said plant having a colour ranging between 33A and 40A measured with reference to the colour patch of The Royal Horticulture Society (RHS) Colour Chart. Preferably, said non-chimeric Pionsettia plant is derived from a somatic embryo produced according to the above described inventive method, more preferably the plant is derived from a somatic embryo that has been produced by using the chimeric cultivars Fiscor Electric (US Plant Patent PP17088). In the inventive non-chimeric poinsettia plant a unique orange bract color has been combined for the first time with a non-chimeric genetic background. The unique combination of a brilliant orange color and the non-chimeric status can be obtained by using different genetic backgrounds as startting material and is not restricted to a specific genetic background which could be considered as a variety. The inventive poinsettia plant can be developed by applying the inventive somatic embryogenesis method disclosed herein to the variety "Fiscor Electric" (US Plant Patent PP17088). Additionally the variety "Harvest Orange" ('PER1180') might serve as starting material as well. The plant produced according to the inventive method will combine a bright orange color with the non-chimeric status.

In an additional embodiment the invention relates to a poinsettia plant obtainable by applying the inventive method as described above to the poinsettia variety "Fiscor Electric" (US Plant Patent PP17088).

### EXAMPLES

The following examples are provided in order to demonstrate and further illustrate certain preferred embodiments and aspects of the present invention and are not to be construed as limiting the scope thereof.

### Example 1: Production of somatic embryos

### Plant material:

Young and soft axillary shoot tips of at least 1,5 cm length were excised from greenhouse-grown stock plants of several poinsettia varieties, e.g. Fiscor Electric (US Plant Patent PP17088) and "Fismars" (US Plant Patent PP14997)

### Sterilisation of plant material:

- Defoliation of small shoots
- Washing of shoots using sterile Aqua dest. (2 x 5 min on a rotary shaker)
- 15 min sterilisation using 3% NaOCI (Potassium-Hypochlorit) + 2 drops Tween 20
- thorough rinsing of shoots using sterile Aqua dest. (3 x 10 min on rotary shaker)
- storage of plant material in a refrigerator at 5-7 C° until usage

### Induction of somatic embryogenesis:

For the induction of somatic embryogenesis and further development of embryogenic callus 1 mm thick subapical stem segments taken from the sterilized axillary shoots were placed on MS-based embryo induction medium 5 (Table 1) as described in the prior art (Preil, W., 1994: In vitro culture of poinsettia. In: Stromme, E (ed), The Scientific Basis of poinsettia Production, 49-55. Agric. Univ. of Norway, As). The experiments were done in petri dishes each containing 25 ml of solid agagar medium. In order to avoid the growth of endophytic bacteria the medium was supplemented with a combination of 200 mg/l cefotaxime and 100 mg/l vancomycin.

**Table 1: MS-based embryo induction medium 5 (Murashige, T. & Skoog, F. (1962) Physiol. Plant. 15, 473 - 497 and Preil, W., 1994: In vitro culture of poinsettia. In: Stromme, E (ed), The Scientific Basis of poinsettia Production, 49-55. Agric. Univ. of Norway, As).**

| BaR/BB | date 13.06.2008 | Fig / Seq |
|---|---|---|
| **Macroelements:** | NH₄NO₃ | 1650 mg/l |
| | CaCl₂ x 2H₂O | 440 |
| | KNO₃ | 1900 |
| | MgSO₄ x 7H2O | 370 |
| | KH₂PO₄ | 170 |
| **Microelements:** | MnSO₄ x 4H₂O | 22.3 mg/l |
| | ZnSO₄ x 7H₂O | 8.6 |
| | H₃BO₃ | 6.2 |
| | CoCl₂ x 6H₂O | 0.025 |
| | KI | 0.83 |
| | CuSO₄ x 5H₂O | 0.025 |
| | Na₂MoO₄ x 2H₂O | 0.25 |
| | FeSO₄ x 7H₂O | 27.8 |
| | Na₂EDTA | 37.3 |
| **Vitamins/Amino acids:** | Glycine | 2.0 mg/l |
| | Inositol | 100 |
| | Nicotinic acid | 0.5 |
| | Pyridoxine HCl | 0.5 |
| | Thiamine HCl | 0.1 |
| **Sucrose:** | | 30 g/l |
| **Hormones** | α-naphthalene acetic acid (NAA) | 0.2 mg/l |
| | 2-isopentenyladenine | 0.1 mg/l |
| **Agar:** | | 7 g/l |
| **pH:** | | 5.7 |

The above describe protocol yielded only an unsatisfying number of somatic embryos for the cultivar Fiscor Electric (US Plant Patent PP17088) and more or less no somatic embryos for the cultivar "Fismars" (US Plant Patent PP14997). Based on the described results one has to conclude that said prior art method is very inefficient and can be applied only to a limited number of poinsettia varieties. Consequently, this method is not applicable for high throughput production or breeding processes.

### Example 2: Improved method for the production of somatic embryos

Surprisingly, it has been found that the efficiency of the protocol used in example 1 could be improve (with respect to the yield rate of somatic embryos as well as to range of *Euphorbia pulcherrima* varieties that can be used as starting material) by using a modified embryo induction medium. In this inventive medium the 2-isopentenyladenine was replaced by Thidiazuron. More surprisingly, it has been found that the use of Thidiazuron alone is not sufficient to improve the prior art method. A specific Thidiazuron concentration must used in order to achieve a higher yield rate and a broader variety spectrum compared to the prior art method.

The impact of the new embryo induction medium (EIM) on the somatic embryo production is described in the following tables. The poinsettia variety "Fismars" (US Plant Patent PP14997) has been used to exemplify the superiority of the inventive method over the prior art. The α-naphthalene acetic acid concentration was kept in the same range, whereas 2-isopentenyladenine was replaced by Thidiazuron (embryo induction medium 10-18). The somatic embryos have been produced as described in example 1. Thidiazuron supplemented to the embryo induction medium with a concentration of at least 0,2 mg/l combined with a similar concentration of NAA induced higher numbers of somatic embryos on subapical stem pieces of poinsettia variety "Fismars" (compared to the prior art methods). Among the hormone combinations and concentrations applied those media were most efficient that contained 0,4 mg/l Thidiazuron (embryo induction medium 12, 15, 18) even if only 0,1 mg/l NAA were added. The best time for inducing somatic embryos in poinsettia is between spring and early summer

**Table 2: Overview on media used for induction of somatic embryos (embryo induction medium 1-18)**

| NAA | 2-isopentenyladenine | | |
|---|---|---|---|
| | 0,05 mg/l | 0,1 mg/l | 0,2 mg/l |
| 0,1 mg/l | EIM 1 | EIM 2 | EIM 3 |
| 0,2 mg/l | EIM 4 | EIM 5 | EIM 6 |
| 0,4 mg/l | EIM 7 | EIM 8 | EIM 9 |
| | | | |

| NAA | Thidiazuron | | |
|---|---|---|---|
| | 0,1 mg/l | 0,2 mg/l | 0,4 mg/l |
| 0,1 mg/l | EIM 10 | EIM 11 | EIM 12 |
| 0,2 mg/l | EIM 13 | EIM 14 | EIM 15 |
| 0,4 mg/l | EIM 16 | EIM 17 | EIM 18 |

All petri dishes were kept under conditions of 24°C and a photoperiod of 16 hours per day. After 3 weeks, the explants were transferred to fresh medium of the same composition for another 3 weeks culture. During this period, callus continuously grew and first embryogenic structures became visible.

**Table 3: Influence of different somatic embryogenesis induction media (EIM) on callus and embryo formation frequency of cultivar "Mars"**

| Initiation Date | Medium | Number of Explants | Growth & Callus formation | | Explants forming somatic embryos number |
|---|---|---|---|---|---|
| | | | after 3 weeks | after 6 weeks | |
| 15.03.2007 | EIM 1 | 81 | 68 | 68 | 0 |
| 10.05.2007 | | 49 | 42 | 21 | 0 |
| 28.06.2007 | | 25 | 24 | 24 | 0 |
| 15.03.2007 | EIM 2 | 81 | 74 | 74 | 0 |
| 10.05.2007 | | 48 | 46 | 46 | 0 |
| 28.06.2007 | | 25 | 25 | 25 | 0 |
| 15.03.2007 | EIM 3 | 81 | 76 | 76 | 0 |
| 10.05.2007 | | 49 | 40 | 40 | 0 |
| 28.06.2007 | | 25 | 16 | 16 | 0 |
| 22.03.2007 | EIM 4 | 49 | 40 | 40 | 0 |
| 24.05.2007 | | 81 | 70 | 70 | 4 |
| 28.06.2007 | | 25 | 0 | 0 | 0 |
| 22.03.2007 | EIM 5 | 49 | 38 | 19 | 0 |
| 24.05.2007 | | 81 | 56 | 56 | 1 |
| 28.06.2007 | | 25 | 24 | 24 | 0 |
| 22.03.2007 | EIM 6 | 50 | 47 | 47 | 0 |
| 24.05.2007 | | 81 | 77 | 77 | 1 |
| 28.06.2007 | | 25 | 24 | 24 | 1 |
| 29.03.2007 | EIM 7 | 51 | 48 | 48 | 0 |
| 31.05.2007 | | 55 | 26 | 0 | 0 |
| 05.07.2007 | | 26 | 26 | 26 | 1 |
| 12.07.2007 | | 51 | 43 | 43 | 0 |
| 29.03.2007 | EIM 8 | 51 | 43 | 43 | 1 |
| 31.05.2007 | | 55 | 23 | 23 | 1 |
| 05.07.2007 | | 25 | 23 | 23 | 0 |
| 12.07.2007 | | 52 | 52 | 52 | 0 |
| 29.03.2007 | EIM 9 | 50 | 46 | 45 | 3 |
| 31.05.2007 | | 55 | 28 | 28 | 1 |
| 05.07.2007 | | 28 | 5 | 5 | 0 |
| 12.07.2007 | | 54 | 53 | 53 | 0 |

| Initiation Date | Medium | Number of Explants | Growth & Callus formation | | Explants forming somatic embryos number |
|---|---|---|---|---|---|
| | | | after 3 weeks | after 6 weeks | |
| 19.04.2007 | EIM 10 | 77 | 70 | 69 | 3 |
| 05.06.2007 | | 42 | 34 | 34 | 0 |
| 05.07.2007 | | 27 | 27 | 27 | 0 |
| 19.07.2007 | | 48 | 48 | 48 | 0 |
| 19.04.2007 | EIM 11 | 76 | 67 | 67 | 2 |
| 05.06.2007 | | 42 | 35 | 35 | 1 |
| 05.07.2007 | | 23 | 23 | 21 | 1 |
| 19.07.2007 | | 48 | 23 | 23 | 2 |
| 19.04.2007 | EIM 12 | 77 | 72 | 67 | 35 |
| 05.06.2007 | | 42 | 34 | 34 | 29 |
| 05.07.2007 | | 15 | 15 | 15 | 15 |
| 19.07.2007 | | 48 | 44 | 44 | 12 |
| 26.04.2007 | EIM 13 | 59 | 51 | 51 | 2 |
| 14.06.2007 | | 54 | 50 | 50 | 0 |
| 19.07.2007 | | 48 | 48 | 48 | 0 |
| 26.04.2007 | EIM 14 | 60 | 54 | 53 | 9 |
| 14.06.2007 | | 54 | 49 | 48 | 5 |
| 19.07.2007 | | 24 | 24 | 24 | 1 |
| 26.04.2007 | EIM 15 | 59 | 53 | 53 | 15 |
| 14.06.2007 | | 54 | 50 | 50 | 0 |
| 26.07.2007 | | 72 | 67 | 65 | 13 |
| 03.05.2007 | EIM 16 | 55 | 54 | 53 | 1 |
| 21.06.2007 | | 33 | 30 | 30 | 0 |
| 07.08.2007 | | 103 | 102 | 102 | 3 |
| 03.05.2007 | EIM 17 | 55 | 54 | 54 | 8 |
| 21.06.2007 | | 33 | 33 | 33 | 0 |
| 26.07.2007 | | 72 | 72 | 72 | 15 |
| 07.08.2007 | | 55 | 55 | 55 | 3 |
| 03.05.2007 | EIM 18 | 55 | 54 | 54 | 13 |
| 21.06.2007 | | 33 | 24 | 18 | 6 |
| 26.07.2007 | | 68 | 55 | 53 | 14 |
| 07.08.2007 | | 59 | 59 | 59 | 6 |

### Example 3: Generation of mature poinsettia plants from somatic embryos produced according to method described in example 2.

After finalizing a period of 2 x 3 weeks on embryo induction medium the developing somatic embryos generated from tissues taken from the varieties 'Fiscor Electric' (US Plant Patent PP17088) and "Fismars" (US Plant Patent PP14997) have been subcultured for additional 4 weeks on a MS-based somatic embryo maturation medium (ERM) to stimulate the germination and further development of embryos. ERM has been identical to EIM except that hormones were replaced by 0.05 mg/l BAP. Callus and globular somatic embryos were removed from auxin containing medium and transferred to maturation medium comprising 0.05 mg/l BAP. This medium was applied independent on type and concentration of hormones of embryo induction medium. Preferably, the culturing step on ERM is repeated several times.

Plants rooted well on hormone-free MS medium and were transferred to the greenhouse.

By using the poinsettia variety "Fiscor Electric" (US Plant Patent PP17088, having a deep orange-red bract color ranging from 43 A to 43 B measured with reference to the colour patch of the RHS Colour Chart) as starting material for the production of somatic embryos and the subsequent regeneration of mature plants, it was possible to produce for the first time a non-chimeric plant with a unique orange bract color, **characterized in that** the intercostal areas of the bracts of said plant having a color ranging between 33A and 40A measured with reference to RHS Colour Chart. This new developed plant (hereinafter referred to as "Fiscor Brillet") combines for the first time the phenotypic feature of having a unique brilliant orange bract color and the genetic feature of being non-chimeric. A combination of said features has not been achieved so far in the prior art.

The Horticultural examination of the 'Fiscor Brillect' plants has confirmed that the combination of characteristics are firmly fixed and retained through successive generations of asexual reproduction. The following traits have been repeatediy observed and are determined to be the basic characteristics of 'Fiscor Brillect'. The phenotype may vary significantly with variations in environment such as temperature, light intensity, and day-length. The following observations, measurements and comparisons describe plants grown in Hillscheid, Germany under greenhouse conditions which approximate those generally used in commercial practice. The plants described were grown in a greenhouse in Hillscheid, Germany, from the summer to winter of 2007. Rooted cuttings were planted in 14 cm pots on July 26, 2007 and were pinched on August 10, which left 7- 8 leaves remaining. The minimum temperature was 18°C. The plants initiated flowers under natural short-day conditions in fall. No black cloth was applied to the greenhouse to simulate short-day conditions. No growth regulator was applied. Observations and measurements were mainly taken in mid December 2007, when the plants were in full flower and about 20-week old. In the following description, color references are made to The Royal Horticultural Society Colour Chart (RHS), 2001. The color references were determined indoors in a north light.
Bract color: Upper surface: RHS 40A, uniform; Lower surface: RHS 43B
Comparision of 'Fiscor Electric' (US PP 17088) and 'Fiscor Brillect'

In comparison/contrast to 'Fiscor Electric', 'Fiscor Brillect' has an even more brilliant and purer orange bract color, generally somewhat weaker (reddish-brown) anthocyanin coloration of petioles and stems.

In order to produce a non-chimeric poinsettia plant with a bract color ranging between 33A and 40A measured with reference to RHS Colour Chart RHS 40A the variety "Fiscor Electric" (see above) can be used. Additionally, the cultivar "Harvest Orange" (registered at the Canadian Plant Breeders' Rights Office under the Application number 07-5962 (denomination name 'PER1180')) could be used as well.

## Claims

1. A method for the production of somatic embryos from poinsettia plant tissue, **characterized in that** an embryo induction medium is used comprising between at least 0.1 and 1 mg/l α-naphthalene acetic acid and between at least 0.2 and 1 mg/l Thidiazuron, with the proviso that if the α-naphthalene acetic acid concentration is below 0.2 mg/l the Thidiazuron concentration is at least 0.4 mg/l.

2. A method according to claim 1, wherein the poinsettia plant tissue employed is young shoot tissue.

3. A method according to claim 1 or 2, **characterized in that** the poinsettia plant is selected from the group consisting of the varieties "Cortez Eletric Fire" (US PP 17088), "Fissilver" (US Plant Patent PP9989), "Nobelstar" (US Plant Patent PP9474), "Fisflirt" (US Plant Patent PP9385), "Fispic" (US Plant Patent PP9371), "Fisbon" (US Plant Patent PP9347), "Fisbla" (US Plant Patent PP9316), "Fispue" (US Plant Patent PP9315), "Fismars Pink" (US Plant Patent PP18866), "Fisdra" (US Plant Patent PP17739), "Fismars Creme" (US Plant Patent PP17658), "Fiscinne" (US Plant Patent PP16964), "Fisholly" (US Plant Patent PP16945), "Fisnovired" (US Plant Patent PP16869), "Fisvinci" (US Plant Patent PP14107), "Kamp Burgundy" (US Plant Patent PP13962), "Fislemon" (US Plant Patent PP13747), "Fiselfi" (US Plant Patent PP13736), "Fisson Jinglit" (US Plant Patent PP13721), "Fisson Beckpink" (US Plant Patent PP13717), "Fismille" (US Plant Patent PP13660), "Fispue White" (US Plant Patent PP13659), "Fismarble Silver" (US Plant Patent PP13174), "Fisson Jingle" (US Plant Patent PP12540), "Fisson Piz" (US Plant Patent PP12539), "Fiscor Hot Pink" (US Plant Patent PP12501), "Fisson Orange" (US Plant Patent PP12500), "Fisnova" (US Plant Patent PP12387), "Fisson Gold" (US Plant Patent PP12298), "Fisgala" (US Plant Patent PP12178), "Fiscor Candy" (US Plant Patent PP11593), "Fisflirt Silver" (US Plant Patent PP11585), "Fisson Marble" (US Plant Patent PP10835), "Fisson White" (US Plant Patent PP10825), "Fiscor Creme" (US Plant Patent PP10824), "Fiswhite silver" (US Plant Patent PP14626), "Fiscor Fire" (US Plant Patent PP12992), "Fiscor dark red" (US Plant Patent PP12723), "Fismond" (US Plant Patent PP14110), "Fismars" (US Plant Patent PP14997), "Fisolymp" (US Plant Patent PP14662), "Fisson dark red" (US Plant Patent PP12722), "Fiselfy pink" (US Plant Patent PP14899), "Fisson" (US Plant Patent PP9365), "Fiscor" (US Plant Patent PP9364), "Fissonosa" (US Plant.Patent PP10182) and "Fiscorosa" (US Plant Patent PP10077).

4. A method for producing a non-chimeric poinsettia plant comprising the steps of:
a) producing somatic embryos according to any one of claims 1 to 3, and
b) growing a mature non-chimeric poinsettia plant from a somatic embryo produced in step a), **characterized in that** the poinsettia plant used in step a) is a chimeric plant.

5. A method according to claim 4, **characterized in that** the poinsettia plant is selected from the group consisting of the varieties Cortez Eletric Fire (US PP 17088), "Fissilver" (US Plant Patent PP9989), "Nobelstar" (US Plant Patent PP9474), "Fisflirt" (US Plant Patent PP9385), "Fispic" (US Plant Patent PP9371), "Fisbon" (US Plant Patent PP9347), "Fisbla" (US Plant Patent PP9316), "Fispue" (US Plant Patent PP9315), "Fismars Pink" (US Plant Patent PP18866), "Fiscinne" (US Plant Patent PP16964), "Fisholly" (US Plant Patent PP16945), "Kamp Burgundy" (US Plant Patent PP13962), "Fisson Jinglit" (US Plant Patent PP13721), "Fisson Beckpink" (US Plant Patent PP13717), "Fispue White" (US Plant Patent PP13659), "Fismarble Silver" (US Plant Patent PP13174), "Fisson Jingle" (US Plant Patent PP12540), "Fisson Piz" (US Plant Patent PP12539), "Fiscor Hot Pink" (US Plant Patent PP12501), "Fisson Orange" (US Plant Patent PP12500), "Fisson Gold" (US Plant Patent PP12298), "Fisflirt Silver" (US Plant Patent PP11585), "Fisson Marble" (US Plant Patent PP10835), "Fiswhite silver" (US Plant Patent PP14626), "Fiscor Fire" (US Plant Patent PP12992), "Fiscor dark red" (US Plant Patent PP12723), "Fisolymp" (US Plant Patent PP14662), "Fisson dark red" (US Plant Patent PP12722), "Fiselfy pink" (US Plant Patent PP14899), "Fissonosa" (US Plant Patent PP10182) and "Fiscorosa" (US Plant Patent PP10077)

6. A method according to claim 4, wherein the chimeric poinsettia plant of step a) has been produced by mutational breeding.

7. A method according to any one of claims 4 to 6, further comprising step c) wherein the plant grown in step b) is infected with a phytoplasma.

8. A method according to claim 7, wherein the phytoplasma infection is obtained by grafting on an infected rootstock or approach grafting.

9. Non-chimeric poinsettia plant, **characterized in that** the intercostal areas of the bracts have a colour ranging from 30A to 40A measured with reference to the colour patch of The Royal Horticulture Society Colour Chart.

10. The progeny of the non-chimeric poinsettia plants of claim 9.

11. Embryo induction medium comprising between at least 0.1 and 1 mg/l α-naphthalene acetic acid and between at least 0.2 and 1 mg/l Thidiazuron, with the provisio, that, if the α-naphthalene acetic acid concentration is below 0.2 mg/l, the Thidiazuron concentration is at least 0.4 mg/l.
